# EUROPÄISCHE PATENTSCHRIFT

(11) **EP 2 888 348 B1**
(45) Veröffentlichungstag und Bekanntmachung des Hinweises auf die Patenterteilung: **03.10.2018**
(21) Anmeldenummer: 13753604.1
(22) Anmeldetag: 20.08.2013
(51) Int. Cl.: A61K 35/28, C12M 1/32, C12N 5/079, C12N 5/0775, B01L 3/00, C12M 1/26, C12M 1/00, C12N 5/00, G01N 33/50, C12M 1/34

(54) **ZELLSELEKTIONSVERFAHREN UND DARAUS GEWONNENE ZELLEN**
CELL SELECTION METHOD AND CELLS OBTAINED THEREFROM
PROCÉDÉ DE SÉLECTION DE CELLULES ET CELLULES AINSI OBTENUES

(30) Priorität: 27.08.2012 DE 102012107879
(43) Veröffentlichungstag der Anmeldung: 01.07.2015
(73) Patentinhaber: Eberhard Karls Universität Tübingen Medizinische Fakultät, 72074 Tübingen (DE)
(72) Erfinder: DANIELYAN, Lusine, 72076 Tübingen (DE); GLEITER, Christoph, 72074 Tübingen (DE); SCHWAB, Matthias, 70180 Stuttgart (DE); SCHÄFER, Richard, 65527 Niedernhausen (DE); BUNIATIAN, Gayane, 72076 Tübingen (DE); PROKSCH, Barbara, 72076 Tübingen (DE)
(74) Vertreter: Witte, Weller & Partner Patentanwälte mbB
(86) Internationale Anmeldenummer: PCT/EP2013/067300
(87) Internationale Veröffentlichungsnummer: WO 2014/033009

(56) Entgegenhaltungen:
- CN-U- 201 495 228
- KR-A- 20100 117 869
- US-B2- 9 096 831
- Sally H. Zigmond ET AL: "Chemotaxis Assays for Eukaryotic Cells" In: "Current Protocols in Cell Biology", 1. Mai 2001 (2001-05-01), John Wiley & Sons, Inc., Hoboken, NJ, USA, XP055081925, ISBN: 978-0-47-114303-1 DOI: 10.1002/0471143030.cb1201s00, Abbildung 12.1.5
- TEN BRINKE A ET AL: "Generation of dendritic cells for immunotherapy is minimally impaired by granulocytes in the monocyte preparation", IMMUNOBIOLOGY, Bd. 211, Nr. 6-8, 14. September 2006 (2006-09-14), Seiten 633-640, XP028020264, URBAN UND FISCHER VERLAG, DE ISSN: 0171-2985, DOI: 10.1016/J.IMBIO.2006.05.012 [gefunden am 2006-09-14]
- Chen, Hong-Chen: "Boyden chamber assay", Methods in Molecular Biology, Bd. 294 28. September 2005 (2005-09-28), Seiten 15-22, XP002714263, Gefunden im Internet: URL:http://www.protocol-online.org/protoco ls/4464.pdf [gefunden am 2013-10-07]
- HUANG YU ET AL: "Evaluation of cancer stem cell migration using compartmentalizing microfluidic devices and live cell imaging.", JOURNAL OF VISUALIZED EXPERIMENTS : JOVE 2011, Nr. 58, E3297, Dezember 2011 (2011-12), Seiten 1-6, XP002714264, ISSN: 1940-087X
- KAWAGUCHI H ET AL: "A new co-culture method utilizing a dual compartment cell-allocation apparatus for observation and identification of synapse formation in vitro", BRAIN RESEARCH, Bd. 594, Nr. 2, 30. Oktober 1992 (1992-10-30), Seiten 284-289, XP024287042, ELSEVIER, AMSTERDAM, NL ISSN: 0006-8993, DOI: 10.1016/0006-8993(92)91136-3 [gefunden am 1992-10-30]

## Beschreibung

Die vorliegende Erfindung betrifft ein Verfahren zur *in vitro* Selektion von zumindest einer Zell-Subpopulation aus einer Original-Zellpopulation eukaryontischer Zellen sowie die mit diesem Verfahren selektierten Zellen und deren Verwendung.

Die zellbasierte Therapie von degenerativen Erkrankungen erwies sich in jüngerer Zeit als interessantes und potentes Therapieverfahren, mit dem unter anderem neurodegenerattve Erkrankungen des zentralen Nervensystems (ZNS), chronische Lebererkrankungen, Herzkrankheiten, Arthritis, oder auch Tumoren behandelt werden sollen. Die zellbasierte Therapie basiert dabei auf dem Ersatz verletzter, geschädigter und/oder in ihrer Regenerationsfähigkeit bzw. normalen Aktivität behinderter oder degenerierter Zellen durch transplantierte Zellen, die entweder natürliche, das heißt unveränderte, native Zellen darstellen, oder aber gentechnisch modifizierte Zellen sind. Der Ersatz dieser degenerierten Zellen durch native bzw. gentechnisch modifizierte Zellen kann dabei beispielsweise durch trophische Faktoren unterstützt werden. Wie bereits erwähnt, werden bei dem Verfahren der Zelltherapie neue Zellen in ein erkranktes oder degeneriertes Gewebe eingeführt, um das aufgrund degenerierter bzw. nicht mehr funktionsfähiger Zellen erkrankte Gewebe zu regenerieren und um dadurch spezifische Krankheiten zu behandeln. Die im Zuge der Zelltherapie gegenwärtig eingesetzten Zellen stellen insbesondere Stammzellen oder Vorläuferzellen von Stammzellen dar, die entweder autolog sind, das heißt von dem Patienten gewonnen wurden, der behandelt werden soll, oder aber aliogen, und damit Zellen darstellen, die von einem Donor erhalten wurden. Die Stammzellen bzw. Vorläuferzellen von Stammzellen differenzieren sich in der Regel in dem Gewebe, in das sie eingeführt werden, zu den ursprünglich im Gewebe vorliegenden Zellen aus, und zwar gerade durch die Einflüsse des umgebenden Gewebes. Alternativ werden gegenwärtig auch reife, funktionale Zellen eingesetzt, oder gentechnisch modifizierte Zellen, die in der Regel zur Produktion einer bestimmten Substanz gentechnisch verändert wurden, welche in dem betreffenden Gewebe benötigt wird oder fehlt.

Insbesondere beim Einsatz von Stammzellen ist es wünschenswert, diejenigen Stammzellen zu selektieren, die besonders aktiv sind bzw. eine erhöhte Potenz besitzen, sich in die reifen Zellen des Gewebes zu differenzieren, in welches die Stammzellen eingeführt werden.

Die gegenwärtig eingesetzten mesenchymalen Stammzellen stellen multipotente adulte Stammzellen dar und besitzen immun-modulatorische Eigenschaften, und können schnell proliferieren. Daher können diese für einen breiten Bereich an Behandlungen eingesetzt werden, einschließlich zum Zwecke einer immun-modulatorischen Therapie, zur Knochen- und Knorpelregeneration, zur Myokardregeneration sowie von neurologischen oder skeleto-muskulären Erkrankungen. Die mesenchymalen Stammzellen können dabei entweder vor deren Differenzierung in das betroffene Gewebe eingeführt werden, oder aber *in vitro* in die betreffenden, erwünschten Zellen ausdifferenziert werden, und nach der Ausdifferenzierung in das verletzte bzw. degenerierte Gewebe eingebracht werden. Die Zellen, also entweder die ausdifferenzierten Zellen, oder aber mesenchymale Stammzellen, integrieren sich dann am Ort der Degeneration und ersetzen
entweder das verletzte oder degenerierte Gewebe, oder wirken einem Voranschreiten der Verletzung/Degeneration durch ihre trophischen und immunmodulatorischen Effekte entgegen, wodurch die Funktion des Organs oder des Gewebes partiell oder gänzlich wiederhergestellt wird.

Wie ebenfalls bereits weiter oben erwähnt, können im Zuge der Zelltherapie auch Zellen zur Regeneration von degeneriertem Gewebe eingesetzt werden, die immunmodulatorisch wirken, um lösliche Faktoren wie beispielsweise Zytokine, Chemokine oder Wachstumsfaktoren zu sekretieren, die in der Umgebung der eingesetzten Zellen ihre Wirksamkeit entfalten.

Insbesondere im Gebiet der Therapie von ZNS-Erkrankungen stellt die Therapie mit adulten Stammzellen einen wichtigen Meilenstein dar. Hierbei liegt das Ziel der Therapie darin, dem Untergang, das heißt also der Degeneration, von Neuronen durch gezielten Schutz oder durch Neubildung entgegenzuwirken.

So wurde die zellbasierte Therapie mittlerweile in fast jeder Art von neurodegenerativen Erkrankungen eingesetzt (siehe beispielsweise Lee et al., 2010, Stern Cells 28: 329-343; Wang et al. , 2006, Journal of Medical Investigation 53: 61 -69), sowie in einer Reihe von akuten ZNS-Erkrankungen, wie traumatische Hirnläsion, Schlaganfall, und darüber hinaus auch bei Tumorerkrankungen.

Trotz der in präklinischen Studien bewiesenen Therapieeffekte von Stammzellen sind die Zellimplantationsansätze mit einer Reihe von Problemen behaftet: So birgt die Implantation von Stammzellen oftmals die Gefahr, dass nicht der erwünschte Phänotyp von Transplantatzellen erlangt und erhalten wird, oder dass die transplantierten Zellen in einer therapeutischen Konzentration im zentralen Nervensystem nicht stabil überleben, oder dass das Empfängergewebe immunologisch reagiert. Darüber hinaus besteht auch die Gefahr, dass die transplantierten Zellen aus dem Transplantationsareal auswandern und somit eine Regeneration des degenerierten Gewebes ebenfalls nicht zustande kommt.

Vor diesem Hintergrund wäre es wünschenswert, eine Subpopulation aus der jeweiligen Stammzellart und dem Entwicklungsstadium selektieren zu können, sowie diese für die zellbasierte Therapie degenerativer Erkrankungen dahingehend evaluieren zu können, ob sie ein besseres Wirksamkeits- und Unbedenklichkeitsprofil besitzen als unselektierte Populationen. Eine solche Selektion wäre sowohl für Tierexperimente als auch für die Therapie von Tier und Mensch, mithin von Säugetieren allgemein, wünschenswert

Entsprechend liegt der vorliegenden Erfindung die Aufgabe zugrunde, ein neues Mittel bereitzustellen, mit welchem Zellen, die erfolgreich in der Zelltherapie eingesetzt werden können, selektiert werden können, bzw. solche Zellen überhaupt bereitzustellen.

Erfindungsgemäß wird diese Aufgabe gelöst durch ein Verfahren zur *in vitro* Selektion von zumindest einer therapeutischen Zell-Subpopulation aus einer Original-Zellpopulation, wobei die Original-Zellpopulation ausgewählt ist aus mesenchymalen Stammzellen und Astroglia, wobei die therapeutische Zell-Subpopulation zur Therapie von Erkrankungen von Geweben oder Organen einsetzbar ist, und wobei das Verfahren die folgenden Schritte aufweist:
a) Aufbringen einer Original-Zellpopulation in ein Ausgangs-Reservoir, das über zumindest ein zumindest eine Öffnung aufweisendes Selektionsmittel mit zumindest einem Selektions-Reservoir verbunden ist, wobei die zumindest eine Öffnung des zumindest einen Selektionsmitteis einen Durchmesser von zwischen 8 µm bis 12 µm aufweist;
b) Aufbringen von zumindest einem Zellkulturmedium in das zumindest eine Selektions-Reservoir, zu welchem die therapeutische Zellpopulation migriert, wobei das Zellkulturmedium ein Zellart-spezifisches Kultivierungsmedium ohne spezifische Zusätze, die das Wachstum bzw. die Differenzierung, Aktivierung und Migration/Chemotaxis der Zellen beeinflussen können, ist;
c) Kultivieren der Original-Zellpopulation für einen Zeitraum von zwischen 15 min und 6 Stunden, innerhalb welchem Zeitraum eine Zell-Subpopulation die zumindest eine Öffnung des Selektionsmittels in Richtung des zumindest einen Selektions-Reservoirs durchwandert;
d) Auswählen und Gewinnen einer Zelle, die ausgewählt ist aus zumindest einer der Zellen, die die zumindest eine Öffnung des Selektionsmittels innerhalb eines Zeitraums der ersten dreißig Minuten als erste bis hundertste Zelle in das zumindest eine Selektions- Reservoir durchwandert haben, zur Gewinnung einer Zell-Subpopulation mit einem im Vergleich zur Original-Zellpopulation erhöhten Migrationspotenzial.

Vorliegend werden ferner durch mittels dem erfindungsgemäßen Verfahren gewonnenen Zellen mit erhöhtem Migrationspotenzial offenbart, insbesondere zu einer Zellart des potentiell zu behandelnden Organs oder Gewebes, sowie deren Verwendung in der Therapie von Erkrankungen, bzw. deren Verwendung in der Herstellung eines Arzneimittels zur Behandlung von Erkrankungen.

Die der Erfindung zugrunde liegende Aufgabe wird auf diese Weise vollkommen gelöst.

Mit dem erfindungsgemäßen Verfahren ist es möglich, Subpopulationen aus Original-Zellpopulationen zu selektieren, die ein erhöhtes Migrationspotenzial zu bestimmter Zellart(en) des potentiell zu behandelnden Organs besitzen und die aufgrund dieses Potenzials therapeutisch besonders potent sind.

Durch das erfindungsgemäße Verfahren können damit bspw. gezielt Subpopulationen von therapeutischen Zellen selektiert und isoliert werden, die sich als besonders potent in der Regeneration von degeneriertem Gewebe, wie beispielsweise Nervengewebe, erwiesen haben.

Durch die Auswahl dieser Subpopulation wird gewährleistet, dass die einzusetzenden bzw. im Rahmen der Zelltherapie einzuführenden therapeutischen Zellen schneller und zielgerichteter zum Zielgewebe migrieren, als dies unselektierte Zellen aus Original-Populationen derselben Zellart tun würden. Dadurch kann eine effektivere und schnellere Regeneration des degenerierten Gewebes bewirkt werden.

Das in Schritt b) vorzulegende Zellkulturmedium wird dabei ohne Zusatz eines der folgenden eingesetzt: eines Cyto-/Chemokins bzw. Wachstumsfaktors, und/oder einer Zellpopulation aus mindestens einer Zellart wie Neuronen, Astro-zyten, immunkompetente Zellen und Tumorzellen des Gehirns, perivaskuläre oder vaskuläre bzw. Bindegewebe- oder Parenchymzellen verschiedener Organe wie Leber, Lunge, Milz, Gastrointestinal- und Urogenitaltrakt-Organe, Haut, Muskel, isoliert aus einem oder mehreren spezifischen Gewebe/Region(en) eines Organs, insbesondere des Hirns, wie bspw. Hippocampus, Bulbus olfactorius.

Vorliegend wird dabei unter einem "Reservoir" jedes Behältnis oder jede Kammer verstanden, das bzw. die zur Aufnahme und Kultivierung von Zellen geeignet ist. Beispielhaft kann für das vorliegende Verfahren ein Behältnis eingesetzt werden, das ausgewählt ist aus Petrischalen, Mikrotiter- bzw. Multiwellplatten, die gegenwärtig üblicherweise im Bereich der Zellkultur und Zelluntersuchung eingesetzt werden. Dabei wird unter dem "Ausgangs-Reservoir" dasjenige Reservoir verstanden, in das die Original-Zellpopulation ein- oder aufgebracht wird, und unter zumindest einem "Selektions-Reservoir" dasjenige/diejenigen Reservoire, in welche die Zell-Subpopulation(en) migriert /migrieren.

Die Original-Zellpopulation wird gemäß dem erfindungsgemäßen Verfahren in das Ausgangs-Reservoir gegeben, von welchem aus die Zell-Subpopulation(en) über die zumindest eine Öffnung des Selektionsmittels innerhalb eines bestimmten Zeitraums, der von der jeweiligen Zell-Subpopulation und den Bedingungen in den Ausgangs-Reservoiren abhängt, in das zumindest eine Selektions-Reservoir migriert/migrieren.

Bei dem erfindungsgemäßen Verfahren können die Schritte a) und b) nacheinander oder gleichzeitig ausgeführt werden; die restlichen Schritte laufen aufeinanderfolgend ab.

Vorliegend wird unter einem "zumindest eine Öffnung aufweisendes Selektionsmittel" jedes Element verstanden, das zumindest eine, vorzugsweise mehrere, Öffnung(en) mit dem wie oben definierten Durchmesser von zwischen 8 µm bis 12 µm
aufweist, mit Hilfe dessen eine Selektion der Original-Zellpopulation erreicht werden kann, indem Zellen der Original-Zellpopulation durch die zumindest eine Öffnung migrieren.

Erfindungsgemäß kann die zumindest eine Öffnung dabei ausgewählt sein aus zumindest einem Kanal mit einem definierten Durchmesser von zwischen 8 µm bis 12 µm, oder zumindest einer Öffnung mit einem Durchmesser von zwischen 8pm bis 12 µm. Vorzugsweise sind dabei mehrere Öffnungen und mehrere Kanäle vorgesehen, die den gleichen bzw. unterschiedliche Durchmesser innerhalb des definierten Bereiches aufweisen können.

Gemäß einer Ausführungsform ist bevorzugt, wenn das Selektionsmittel eine Poren-aufweisende Membran ist. Bei dieser Ausführungsform des Selektionsmittels ist die zumindest eine Öffnung eine Pore, bzw. mehrere Poren, über welche die Zell-Subpopulation in die Selektions-Reservoire migriert.

Gemäß einer anderen Ausführungsform ist bevorzugt, wenn das zumindest eine Öffnung aufweisende Selektionsmittel zumindest ein Kanal mit dem definierten Durchmesser ist, über welchen das Ausgangs-Reservoir und das zumindest eine Selektions-Reservoir miteinander in Fluidverbindung stehen. Bei Vorliegen mehrerer Selektions-Reservoire ist das Ausgangs-Reservoir mit diesen jeweils über entsprechende Kanäle verbunden.

Ferner wird vorliegend unter "therapeutische Zell-Subpopulation" oder unter "therapeutische Zelle" jede Subpopulation bzw. jede Zelle verstanden, die gezielt und effektiv für den Einsatz in der Zell-Therapie wie eingangs erläutert eingesetzt werden kann und somit eine Regeneration des zu behandelnden Gewebes oder Organs, bspw. eines verletzten oder degenerierten Gewebes oder Organs bewirkt. Definitionsgemäß umfasst eine "therapeutische Zell-Subpopulation" oder "therapeutische Zelle" daher zumindest eine Zelle oder Zelltyp, wie bspw. adulte mesenchymale Stammzellen, die an das degenerierte oder verletzte Gewebe oder Organ gebracht werden oder migrieren, das mit der therapeutischen Zelle/Zell-Subpopulation behandelt werden soll. Die therapeutische Zelle/Zell-Subpopulation kann dabei von jeder eukaryon- tischen Quelle stammen, insbesondere von Säugetieren, und ist eine menschliche Zelle/Zell-Subpopulation von mesenchymalen Stammzellen oder Astroglia, und sich in unterschiedlichen Differenzierungs-Stadien befinden, unter der Voraussetzung, dass sie dazu in der Lage sind, die zu behandelnde Erkrankung zu verhindern oder zur lindern, zu deren Zell-Therapie sie eingesetzt werden sollen. Es versteht sich, dass die therapeutische Zelle/Zell-Subpopulation autolog sein kann, d.h. von dem Patienten stammen kann, der behandelt werden soll, oder aber heterolog, d.h. von einem Spender gewonnen wurde. Autologe Zellen werden in der Regel von dem Patienten gewonnen, ex vivo manipuliert und in den zu behandelnden Patienten wieder eingebracht.

Die eukaryontische Original-Zellpopulation und/oder die Zell-Subpopulation, die ausgewählt sind aus mesenchymalen Stammzellen und Astroglia, kann von jedem Organ oder Gewebe, vorzugsweise eines humanem Organs oder Gewebes, adult oder fötal, gewonnen werden, einschließlich Knochenmark, neurale Gewebe, einschließlich Hippocampus-Gewebe, und Gewebe der subventrikulären Zone, des Zerebellums, des Kortex, des basalen Vorderhirns, des ventralen Mesencephalon und des Locus ceruleus.

Gemäß einer bevorzugten Ausführungsform des erfindungsgemäßen Verfahrens ist die Original-Zellpopulation ausgewählt aus mesenchymalen Stammzellen und Astroglia. Gemäß einer bevorzugten Ausführungsform sind embryonale Zellen nicht umfasst.

Adulte mesenchymale Stammzellen sind Vorläuferzellen des Bindegewebes und besitzen ein hohes Differenzierungspotenzial; sie tragen zur Aufrechterhaltung und Regeneration des Stütz- und Bindegewebes, wie Knochen, Knorpel, Muskel, Bändern, Sehnen und Fettgewebe bei und unterstützen Wachstum und Entwicklung der Vorläuferzellen des Blutes im Knochenmark. MSC aus unterschiedlichen Geweben (Knochenmark, Knorpel, Fettgewebe, Muskel, Lebergewebe, Blut, Amnionflüssigkeit) lassen sich kultivieren und *in vitro* in unterschiedliche Gewebe ausdifferenzieren. Ausdifferenzierte MSC sind darüber hinaus in der Lage, sich in ein anderes Gewebe zu transformieren und sich an neuartige Umgebungsbedingungen anzupassen.

Astroglia stellen charakteristische Sternen-förmige Gliazellen des Gehirns und Rückenmarks dar. Zu Ihren Aufgaben zählen die biochemische Unterstützung von Endothelzellen, die die Blut-Hirn-Schranke bilden, ferner die Bereitstellung von Nährstoffen an das neuronale Gewebe, die Aufrechterhaltung der extrazellulären Ionenbilanz, und sie besitzen eine Rolle bei der Reparatur des Gehirns nach traumatischen Verletzungen.

Gemäß des erfindungsgemäßen Verfahrens weist die zumindest eine Öffnung des Selektionsmittels eine Porengröße von zwischen 8 und 12 µm auf.

Bei der Auswahl dieser Porengröße bzw. Kanäle mit diesem Durchmesser werden besonders migrationsaktive Zellen isoliert.

Gemäß dem erfindungsgemäßen Verfahren beträgt der Zeitraum in Schritt c) zwischen 15 min und 6 Stunden beträgt.

Innerhalb dieses Zeitraums konnten in eigenen Versuchen Zellarten mit erhöhtem Migrationspotenzial selektiert werden.

Dabei wird vorliegend unter "Migrationspotenzial" die Eigenschaft von Zellen verstanden, sich in bestimmte Richtungen an spezifische Orte bewegen zu können. Entsprechend bewegen sich/wandern Zellen mit erhöhtem Migrationspotenzial schneller als Zellen mit einem durchschnittlichen oder niedrigeren Migrationspotenzial.

Es ist bekannt, dass die Migration von Zellen durch spezifische externe Signale beeinflusst werden; dieser Prozess wird auch als Chemotaxis bezeichnet.

Es ist ferner bekannt, dass die Migrationsrichtung einer Zelle durch Tropismus (eine zielgerichtete Migration zu spezifischen Zell- und Gewebearten) vorgegeben sein kann. Diese Zell-bzw. Gewebearten können sich sowohl im normalen physiologischen als auch im pathologischen Zustand befinden. Die pathologische Zustände können sowohl Pathologieübergreifend, wie bspw, Entzündung, Zelltod, inklusive Apoptose oder Nekrose, Hypoxie, Ischämie, Entartung, Neubildung, Ödeme, mechanische Schädigung, als auch durch spezifische Marker der jeweiligen Erkrankung, wie bspw. Amyloid-beta bei Morbus Alzheimer, Kollagen I, III, IV and VI bei Lebererkrankungen, gekennzeichnet sein.

Erfindungsgemäß werden dabei diejenigen Zellen selektiert, die sich indem definierten Zeitraum als erste durch die zumindest eine Öffnung des Selektionsmittels in das Zellkulturmedium des Reservoirs bewegen, wobei bevorzugt zumindest eine der Zellen ausgewählt wird, die sich in einer der ersten bis hundertste Zelle, die sich durch die zumindest eine Öffnung des Selektionsmittel bewegt haben, ausgewählt wird. Entsprechend ist dabei nicht ein bestimmter Zeitraum relevant, sondern die Reihenfolge, in der sich die Zellen durch die zumindest eine Öffnung des Selektionsmittels bewegen: Die Zellen, die zu den ersten einhundert Zellen zählen, die durch die Öffnung des Selektionsmittels, also den Kanälen oder der Poren der Membran gewandert sind, sind dabei von besonderem Interesse für die Selektion.

Insbesondere sind dabei Zellen bevorzugt, die sich unter den ersten fünfzig Zellen befinden, die durch die zumindest eine Öffnung des Selektionsmittels migriert sind.

Gemäß dem erfindungsgemäßen Verfahren sind dabei ferner diejenigen Zellen zur Selektion vorgesehen, welche innerhalb eines Zeitraums der ersten dreißig Minuten durch die zumindest eine Öffnung des Selektionsmitteis migrieren.

Das vorliegend zu verwendende Material des Selektionsmittels, also bspw. der Membran bzw. des Kanals, ist dabei ein Material, das im Gebiet der Zellbiologie bzw., in der *in vitro* Biotechnologie eingesetzt werden kann, und das gegenüber den Zellen inert ist, mithin also die Zelle weder durch ihre Beschaffenheit noch durch ihre chemische Zusammensetzung beeinflusst, sondern lediglich zumindest eine Öffnung, insbesondere Poren/Kanäle mit bestimmter bzw. unterschiedlicher Größe aufweist, durch welche Zellen migrieren können.

Membranen, die im vorliegenden erfindungsgemäßen Verfahren als Selektionsmittel eingesetzt werden können, sind beispielsweise käuflich erhältlich von der Firma Greiner Bio-one, Deutschland, und bestehen im Wesentlichen aus Materialien wie Polyethylenterephthalat oder Polycarbonat. Die Kanäle als Alternative zu Membranen können aus Glas, Quarzglas, Kunststoffe (z.B. Polystyrol) oder ähnlichen Materialien bestehen oder diese Materialien aufweisen, deren Struktur die optische Beobachtung lebender Zellen durch Mikroskopie erlaubt. Erfindungsgemäß dient dabei die Membran als eine Art Trennschicht zu zumindest dem ersten und dem zweiten Reservoir.

Bei einem Einsatz zumindest eines Kanals als Selektionsmittels zwischen dem ersten und dem zweiten Reservoir, dient der zumindest eine Kanal als eine Verbindung zwischen den beiden Reservoiren.

Erfindungsgemäß können in bevorzugten Ausführungsformen auch zwei, drei, vier, fünf oder sechs oder mehr Selektions-Reservoire vorgesehen sein, welche jeweils durch ein zumindest eine Öffnung aufweisendes Selektionsmittel von dem ersten Ausgangs-Reservoir, in das die Original-Zellpopulation eingebracht wird, getrennt sind.

Gemäß einer weiteren bevorzugten Ausführungsform des erfindungsgemäßen Verfahrens werden zumindest 2, 3, 4, 5, oder 6 in Reihe nachgeschaltete Reservoire zur Selektion eingesetzt. Bei dieser Struktur, in der zwei oder mehrere Reservoire getrennt in einem Behältnis (z.B. Petri-Schale) eingebracht und bspw. über einen oder mehrere Kanälen verbunden sind, findet die Zellselektion durch Migration aus dem Ausgangs-Reservoir in die anderen durch ein Zwischenreservoir statt, aus welchem die Zelle selektiert wird, bevor sie das Zielreservoir erreicht.

Die oben erwähnten Ausführungsformen haben den Vorteil, dass mittels der Bereitstellung mehrerer Selektions-Reservoire Zell-Subpopulationen - und hieraus Zellen - selektiert werden können, die sich in unterschiedlichen Selektions-Reservoiren unter Krankheits-nachahmenden Bedingungen behaupten können.

Bekannt ist der Tropismus mancher Stammzellen zu bestimmten Hirnarealen, wie er bspw. für bestimmte Neuronen beschrieben wurde (siehe bspw. Wray *et al*., 1989 und Lois *et al*., 1996), oder die läsions-/inflammations- oder degenerationsgerichtete Migration von Stammzellen in vielen neurodegenerativen Modellen sowie Tumormodellen (Kelly *et al*., 2004, Park et *al*., 2009).

Bei dem erfindungsgemäßen Verfahrens ist dabei das Zellkulturmedium, das in dem zumindest einen Reservoir, insbesondere in dem zumindest einen Selektions-Reservoir vorliegt, ein Zellart-spezifisches Kultivierungsmedium ohne spezifische Zusätze, die das Wachstum bzw. Differenzierung, Aktivierung und Migrati-on/Chemotaxis der Zellen beeinflussen können, wie bspw. SDF-1 a/CXCL12, Cdc42, Integrine, Aktivatoren der Wnt-Signalkaskade, Komponenten der extrazellulären Matrix). Zu solchen gehören insbesondere Hormone, Zytokine, Wachstumsfaktoren (nerve growth factor [NGF], basic fibroblast growth factor [bFGF], epidermal growth factor [EGF], brain-derived neurotrophic factor [BDNF], insulin-like growth factor [IFG-1], platelet derived growth factor [PDGF], transforming growth factor-b [TGF-b]) und andere Moleküle, die Zellen in ihrem Aktivierungszustand beeinflussen können, wie z.B. Entzündungsmediatoren, bspw. Interleukine wie IL-1 ,2,6, oder Peptide mit entzündlicher/toxischer bzw. anti-inflammatorischer bzw. anti-apoptotischer Wirkung wie IL-10, 4,5, Erythropoietin.

Beispielhaft kann das Zellkulturmedium DMEM (Dulbecco's Modified Eagle's Medium), eingesetzt werden, das bei einer Reihe verschiedener Anbieter auf dem Markt erhältlich ist. DMEM stellt im Gebiet der Zellkultivierung ein üblicherweise eingesetztes Medium dar, das neben Aminosäuren und Salzen in der Regel auch Glucose und Vitamine enthält.

Gemäß einer Alternative der erwähnten Ausführungsform des erfindungsgemäßen Verfahrens kann das Zellkulturmedium auch bspw. DMEM ohne Glucose oder mit LPS (Lipopolysacchariden) sein.

Vorliegend ist nicht zur Erfindung gehörend offenbart, dass Zelltypen im Kulturmedium in dem zumindest einen Selektions-Reservoir, in das die Zell-Subpopulationen migrieren, bereits vor der Migration der Zell-Subpopulation in dem zumindest einen Selektions-Reservoir vorliegen können. Diese Zelltypen können dabei derart ausgewählt sein, dass durch deren Vorliegen in dem zumindest einen Selektions-Reservoir Bedingungen nachgeahmt werden, wie sie für bestimmte degenerative Krankheiten spezifisch sind. Diese Zellen können dabei ausgewählt sein aus zumindest einem der folgenden: Zellen, mit weichen *in vitro* Modelle für neurodegenerative Erkrankungen nachgeahmt werden können; Zellen, mit welchen *in vitro* Modelle für Tumore nachgeahmt werden können; Zellen, mit welchen *in vitro* Modelle für Entzündungserkrankungen nachgeahmt werden können; Zellen, mit welchen *in vitro* Modelle für traumatische oder oxidative Stresszustände nachgeahmt werden können. So kann bspw. mit APP (Amyloid Precursor Protein)-überexprimierenden Zellen die Bedingungen der Alzheimer-Erkrankung nachahmt werden, mit alpha-synculein-exprimierenden Zellen die Bedingungen der Parkinson-Erkrankung, oder aber allgemein Tumor oder Traumata-Bedingungen, die durch einen Sauerstoffmangel (Hypoxie), oxidativen Stress und/oder Entzündungen charakterisiert sind. Verschiedene Zellmodelle für Krankheiten von Säugetieren, insbesondere des Menschen, sind im Gebiet der Zellkulturforschung bekannt, und können entsprechend zum Einsatz im vorliegenden Verfahren gebracht werden. Durch das Vorlegen von Zellen in dem zumindest einen Selektions-Reservoir kann einerseits erreicht werden, dass diejenigen Zell-Subpopulationen aus der Original-Zellpopulation, welche von dem Ausgangs-Reservoir über das zumindest eine zumindest eine Öffnung aufweisende Selektionsmittel in das zumindest eine Selektions-Reservoir wandern, durch die durch die Zelltypen vorgegebenen Bedingungen in ihrer Differenzierung bzw. Aktivierung beeinflusst werden können. So kann zur Auswahl einer auf eine ganz bestimmte Organ- bzw. Geweberegion spezialisierten Zell-Subpopulation die Zellmischung bzw. Original-Zellpopulation derart in das Ausgangs-Reservoir aufgebracht, dass die Original-Zellpopulation durch Vorlage mehrerer unterschiedlicher Zelltypen und/oder Substanzen in jeweils unterschiedlichen Selektions-Reservoiren, die durch Öffnungen aufweisende Selektionsmittel von dem Ausgangs-Reservoir getrennt sind, die Wahl zwischen mehreren "Endzielen" haben. Die Zell-Subpopulationen lassen sich dadurch ganz spezifisch selektieren, da sich mit dem erfindungsgemäßen Verfahren Subpopulationen bilden, die zu unterschiedlichen Selektions-Reservoiren migrieren und dadurch unterschiedliche Spezifitäten haben.

Vorliegend ist nicht zur Erfindung gehörend offenbart, dass , dem Zellkulturmedium zur Induktion der Wanderung der Zellen durch die zumindest eine Öffnung des Selektionsmittels zumindest eine weitere Substanz zugesetzt werden kann, die ausgewählt ist aus Chemoattraktanten, pharmakologisch wirksamen Substanzen, Hormonen oder Wachstumsfaktoren. Der Einsatz dieser Substanzen kann in Abhängigkeit des jeweils gewünschten Zell-Subpopulations-Typs ausgewählt werden, und im Hinblick auf das zu regenerierende Organ oder Gewebe. Z.B. Amyloid beta für das ZNS-Gewebe bei M. Alzheimer, HIF1 , Entzündungsmediatoren (Interleukine) für Tumor und ischämische Läsionen des ZNS und anderer Organe. Auch dadurch können Organ-spezifische und Gewebe-spezifische Zellarten von Säugetieren als Zellpopulationen mit hohem Migrationspotenzial selektiert werden. Ferner kann durch die Anwendung von degenerativen Bedingungen in einem Selektions-Reservoir - sei es durch ein Zellmodell oder durch eine entsprechende, ggf. ohne Zusatzstoffe versorgte, Zusammensetzung des Zellkulturmediums - können andererseits besonders robuste Zell-Subpopulationen, bspw. MSC, selektiert werden.

Es versteht sich in diesem Zusammenhang, dass bei Vorliegen mehrerer Selektions-Reservoire in diesen unterschiedliche Bedingungen und/oder Zelltypen vorliegen können. Auf diese Weise können die Zell-Subpopulationen im Hinblick auf die Unterschiedlichen Bedingungen oder Zelltypen selektiert werden, je nach dem in welche der verschiedenen Selektions-Reservoire die Zell-Subpopulation migriert.

Vorliegend werden auch die mit dem erfindungsgemäßen Verfahren gewonnenen Zell-Subpopulation, bzw. Zelle mit erhöhtem Migrationspotenzial offenbart, die insbesondere zur Therapie von Erkrankungen von Säugetieren, bzw. zur Herstellung eines Arzneimittels zur Behandlung von Erkrankungen von Säugetieren, insbesondere des Menschen eingesetzt werden können.

Die Zellen bzw. die Original-Zellpopulationen können dabei humanen Ursprungs sein, können aber im Hinblick auf jedes Säugetier ausgewählt werden und für das Säugetier eingesetzt werden, von dem die Original-Zellpopulation gewonnen wurde.

Die mit dem erfindungsgemäßen Verfahren gewonnenen Zellen mit hohem Migrationspotenzial können eingesetzt werden zur Therapie von Erkrankungen, die ausgewählt sind aus Tumoren, Traumata, Läsionen, degenerative Erkrankungen des Gehirns, der Leber, Niere, Lungen, des Herzens, Gastrointestinaltrak-tes, der Muskeln, Haut, Blut und Knochen.

Da die mit dem erfindungsgemäßen Verfahren selektierten Zellen bzw. Zell-Subpopulationen sich durch eine hohe Migrationsfähigkeit bzw. ein hohes Migrationspotenzial auszeichnen, sind diese insbesondere für den Einsatz bei Zell-Therapien geeignet, bei denen die im Rahmen der Zell-Therapie einzusetzenden Zellen nicht unmittelbar in der zu behandelnden Organ- oder Gewebe-Region eingesetzt werden können, bspw. aus anatomischen Gründen. Dies ist insbesondere bei Erkrankungen vorteilhaft, wo es wichtig ist, dass die Zellen auch an periphere Regionen oder in das ZNS gelangen, um dort das degenerierte Gewebe zu ersetzen. Damit wird vorteilhafterweise erreicht, dass die therapeutisch eingesetzten Zellen nicht an Stellen im Gewebe eingesetzt bzw. appliziert werden, die in der Nähe oder neben den erkrankten bzw. degenerierten Zellen liegen (z.B. intravenöse, intraarterielle, intrathekale Applikation bei ZNS Erkrankungen).

Entsprechend können die über das erfindungsgemäße Verfahren selektierten Zellen mit hohem Migrationspotenzial gezielt für die Therapie der weiter oben erwähnten Erkrankungen eingesetzt werden.

Die adulte Zelle mit hohem Migrationspotenzial kann dabei eine mesenchymale Stammzelle oder Astroglia sein. Gemäß einer bevorzugten Ausführungsform sind embryonale Zellen nicht umfasst.

Vorliegend wird auch eine pharmazeutische Zusammensetzung offenbart, die zumindest eine der wie oben beschriebenen erfindungsgemäß selektierten Zellen aufweist, in Kombination mit zumindest einem pharmazeutisch akzeptierbaren Träger.

Dabei wird unter einem pharmazeutisch akzeptierbaren Träger jeder Hilfsstoff, Zusatz oder pharmazeutische Träger verstanden, der üblicherweise im Gebiet der medizinischen Zell-Therapie eingesetzt wird, und der die Verabreichung der selektierten Zellen vereinfacht oder möglich macht.

Die einzusetzende therapeutisch wirksame bzw. therapeutisch effektive Menge der einzusetzenden, mit dem erfindungsgemäßen Verfahren selektierten Zellen mit erhöhtem Migrationspotenzial ist dabei diejenige Menge, durch welche die Regenerierung des degenerierten bzw. geschädigten Gewebe zu bewirken. Die genaue effektive Menge für einen Patienten hängt dabei insbesondere von dessen Größe und Gesundheitszustand, der Art und dem Ausmaß der Erkrankung ab.

Die pharmazeutische Zusammensetzung kann einem Patienten in einer Vielzahl von Formen verabreicht werden, die den gewählten Weg der Verabreichung, nämlich parenteral, oral, intraperitoneal, transdermal etc. angepasst sind.

Üblicherweise werden die Zellen für die Zell-Therapie transplantiert. Bevorzugt sind dabei insbesondere nichtinvasive Applikationen, wie bspw. eine intranasale Applikation bei Erkrankungen des ZNS.

Wie ferner weiter oben erwähnt, kann die pharmazeutische Zusammensetzung auch pharmazeutisch akzeptierbare Träger, Verbindungsmittel, Verdünnungsmittel, Exzipienten oder Adjuvantien aufweisen. Die Wahl eines pharmazeutischen Trägers, Exzipienten oder anderen Hilfsmittels kann im Hinblick auf den beabsichtigten Verabreichungsweg und die standardisierte pharmazeutische Praxis vorgenommen werden. Als pharmazeutisch akzeptierbare Träger können Lösungsmittel, Streckmittel oder andere flüssige Bindemittel wie Dispersions- der Suspensionshilfsmittel, oberflächenaktive Agentien, isotonische Wirkstoffe, Deckungsmittel oder Emulgatoren, Konservierungsmittel, Umhüllungsmittel, feste Bindestoffe oder Gleitmittel eingesetzt werden, je nachdem, was für die jeweilige Dosierung am besten geeignet ist und zugleich mit der erfindungsgemäßen Kombination kompatibel ist. Eine Übersicht über solche zusätzliche Inhaltsstoffe findet sich bspw. in A. Kibbe: Handbook of Pharmaceutical Excipients, 3rd Edition, 2000, American Pharmaceutical Association and Pharmaceutical Press.

Vorliegend kann ein Zellkultur-Reservoirsystem, das ein Ausgangs-Reservoir, zumindest ein zumindest eine Öffnung aufweisendes Selektionsmittel, sowie zumindest ein Selektions-Reservoir aufweist, wobei die Öffnung einen Durchmesser von zwischen 8 und 12 µm aufweisen, in der erfindungsgemäßen Selektion von zumindest einer Zell-Subpopulation mit erhöhtem Migrationspotenzial verwendet werden.

Wie weiter oben erwähnt wurde im Zuge der vorliegenden Erfindung zum ersten Mal die Eignung eines solchen Systems zur gezielten Selektion von Zellen, wie oben beschrieben, getestet und erfolgreich eingesetzt.

Weitere Vorteile ergeben sich aus der nachstehenden Beschreibung sowie den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Figuren dargestellt und werden in der nachfolgenden Beschreibung näher erläutert. Es zeigen:
Fig. 1 Eine Ausführungsform der erfindungsgemäßen Verwendung des Reservoir-Systems mit einer beispielhafte Anordnung von Zellkultivierungs-Reservoiren mit Kanälen (A und B) oder Membranen (C und D) als Öffnungen bzw. Selektionsmittel, zur Durchführung des erfindungsgemäßen Verfahrens; mit einer Selektion der Subpopulation im Hinblick auf bestimmte Hirnregionen (A und C), bzw. im Hinblick auf toxische, krankheitsrelevante Bedingungen (B und D); und
Fig. 2 Ein Diagramm, das die Migrationsfähigkeit von unselektierten MSC bzw. Astroglia im Vergleich zu einer selektierten Subpopulation zeigt.

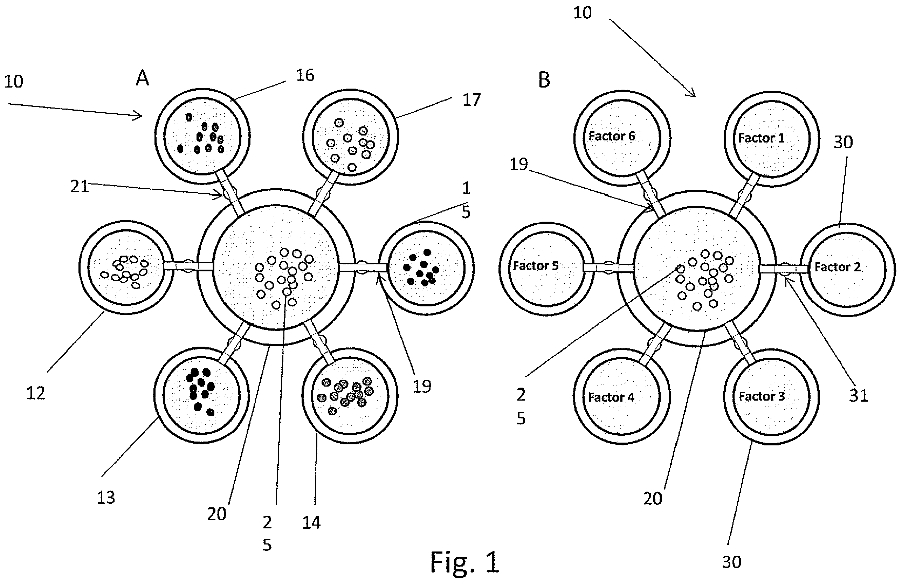

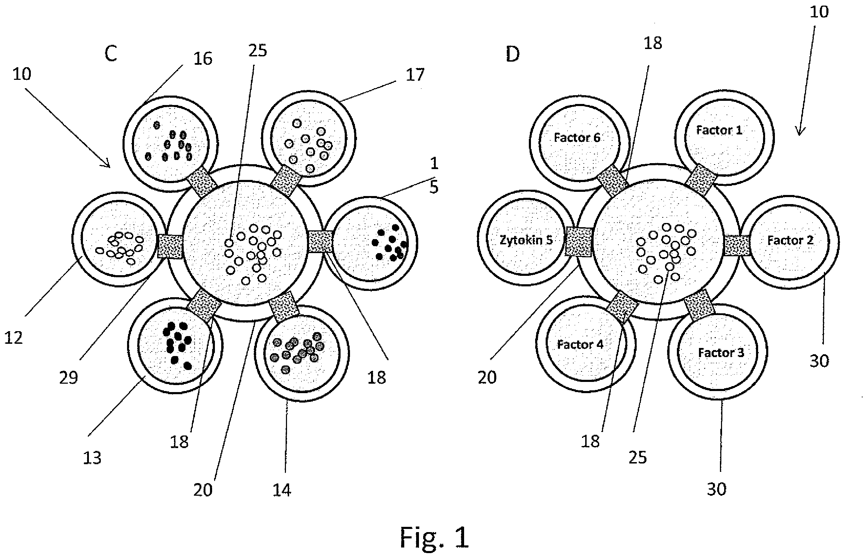

In Fig. 1A ist ein schematisches Reservoirsystem 10 gezeigt, mit sechs Selektions-Reservoiren 12, 13, 14, 15, 16, 17, die kreisförmig um ein zentrales Ausgangs-Reservoir 20 angeordnet sind. !n den Selektions-Reservoirs sind verschiedene Zelltypen vorgelegt, die in dem in Fig. 1A und C gezeigten Beispiel verschiedene Zelltypen des Gehirns sind. Als Selektionsmittel ist in Fig. 1C und 1D eine Membran 18 vorgesehen, die als Öffnungen Poren 29 aufweist, die in der Fig. 1C und D durch ein punktförmiges Füllungsmuster dargestellt sind. In Fig. 1A und 1B sind als Selektionsmittel Kanäle 19 vorgesehen, über welche das Ausgangs-Reservoir 20 mit Selektions-Reservoiren 30 verbunden ist. Das System 10 in Fig. 1A und 1B weist ferner Zwischenreservoire 21 auf.

Über die Poren 29 der Membran 18 oder Kanäle 9 wandern Zellen aus einer Zellmischung 25 aus dem Ausgangs-Reservoir 20 in Richtung einzelner Selektions-Reservoire 12, 13, 14, 15, 16, 17, so dass nach einem bestimmten Zeitpunkt in unterschiedlichen Zwischenreservoiren 21 verschiedene Subpopulationen von bspw. mesenchymalen Stammzellen vorliegen, die eine jeweilige Spezifität für die betreffenden Zelltypen des Gehirns haben. Die dadurch aus den Zwischenreservoiren 21 selektierten Zellen können dann spezifisch für Erkrankungen des Teils des Gehirns eingesetzt werden, für das sie sich spezifisch erwiesen haben.

In Fig. 1B und D ist ein ähnlicher Aufbau für ein Reservoirsystem gezeigt, das nicht erfindungsgemäß eingesetzt wird, wobei bei der in Fig. 1B und D gezeigten Ausführungsform in den verschiedenen Selektions-Reservoiren 30 unterschiedliche Zytokine vorgelegt sind. Die durch die Poren der Membran 18 in D bzw. Kanäle in B zu den einzelnen und unterschiedlichen Selektions-Reservoiren 30 wandernden Zellen 22 können nach deren Selektion in den unterschiedlichen Selektions-Reservoiren 30 (in D) bzw. in den Zwischenreservoiren 31 (in B) dann im Hinblick auf dasjenige Milieu zur Therapie ausgewählt werden, mit welchem eine spezifische Krankheiten nachgeahmt wurde.

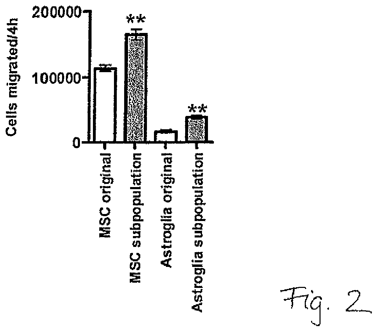

in Fig. 2 sind die Ergebnisse zu Untersuchungen der Migrationsfähigkeit verschiedener Zelltypen dargestellt. Dabei wurde einmal zwischen einer Original-Population von MSC und einer selektierten MSC-Subpopulation unterschieden, sowie zwischen Original-Astroglia und einer selektierten Astroglia-Subpopulation.

Der Vergleich der Migrationsgeschwindigkeit über einen Zeitraum von 4 Stunden zeigt, dass die selektierten Subpopulationen der MSC und der Astroglia jeweils schneller zur Primärkultur migrierten als die jeweiligen Originalpopulationen.

## Patentansprüche

1. Verfahren zur *in vitro* Selektion von zumindest einer therapeutischen Zell-Subpopulation aus einer Original-Zellpopulation, wobei die Original-Zellpopulation ausgewählt ist aus mesenchymalen Stammzellen und Astroglia, wobei die therapeutische Zell-Subpopulation zur Therapie von Erkrankungen von Geweben oder Organen einsetzbar ist, und wobei das Verfahren die folgenden Schritte aufweist:
a) Aufbringen einer Original-Zellpopulation in ein erstes Ausgangs-Reservoir (20), das über zumindest ein zumindest eine Öffnung (19; 29) aufweisendes Selektionsmittel (18; 19) mit zumindest einem zweiten Selektions-Reservoir (12, 13, 14, 15, 16, 17; 30) verbunden ist, wobei die zumindest eine Öffnung (19; 29) des zumindest einen Selektionsmittels (18; 19) einen Durchmesser von zwischen 8µm bis 12µm aufweist;
b) Aufbringen von zumindest einem Zellkulturmedium in das zumindest eine Selektions-Reservoir (12, 13, 14, 15, 16, 17; 30), zu welchem die therapeutische Zell-Subpopulation migriert, wobei das Zellkulturmedium ein Zellart-spezifisches Kultivierungsmedium ohne spezifische Zusätze, die das Wachstum bzw. die Differenzierung, Aktivierung und Migration/Chemotaxis der Zellen beeinflussen können, ist;
c) Kultivieren der Original-Zellpopulation für einen Zeitraum von zwischen 15 min und 6 Stunden, innerhalb welchem Zeitraum eine Zell-Subpopulation die zumindest eine Öffnung (19; 29) des Selektionsmittels (18; 19) in Richtung des zumindest einen Selektions-Reservoirs (12, 13, 14, 15, 16, 17; 30) durchwandert;
d) Auswählen und Gewinnen einer Zelle, die ausgewählt ist aus zumindest einer der Zellen, welche die zumindest eine Öffnung (19; 29) des Selektionsmittels (18; 19) innerhalb eines Zeitraums der ersten dreißig Minuten als erste bis hundertste Zelle in das zumindest eine Selektions-Reservoir (12, 13, 14, 15, 16, 17; 30) durchwandert haben, zur Gewinnung einer Zell-Subpopulation mit einem im Vergleich zur Original-Zellpopulation erhöhten Migrationspotenzial.

2. Verfahren nach Anspruch 1, **dadurch gekennzeichnet, dass** mindestens 2, 3, 4, 5, oder 6 Selektions-Reservoire (12, 13, 14, 15, 16, 17; 30) vorgesehen sind.

## Claims

1. Method for the *in vitro* selection of at least one therapeutic cell subpopulation from an original cell population, wherein the original cell population is selected from mesenchymal stem cells and astroglia, wherein the therapeutic cell subpopulation can be used for the treatment of diseases of tissues or organs, and wherein the method has the following steps:
a) Addition of an original cell population to a first starting reservoir (20), which is connected via at least one selection means (18;19) having at least one opening (19; 29) to at least one second selection reservoir (12, 13, 14, 15, 16, 17; 30), wherein the at least one opening (18; 29) of the at least one selection means (18; 19) has a diameter of 8 µm to 12 µm;
b) Addition of at least one cell culture medium to the at least one selection reservoir (12, 13, 14, 15, 16, 17; 30) to which the therapeutic cell subpopulation migrates, wherein the cell culture medium is a cell type-specific culture medium without specific additives that can influence the growth or differentiation, activation, and migration/chemotaxis of the cells;
c) Culturing of the original cell population for a period of 15 min to 6 hours, within which period a cell subpopulation migrates through the at least one opening (19; 29) of the selection means (18; 19) towards the at least one selection reservoir (12, 13, 14, 15, 16, 17; 30);
d) Selection and obtaining of a cell, which is selected from at least one of the cells that have migrated, within the first thirty minutes, as the first to the one hundredth cell through the at least one opening (19; 29) of the selection means (18; 19) into the at least one selection reservoir (12, 13, 14, 15, 16, 17; 30), in order to obtain a cell subpopulation with an elevated migration potential as compared to the original cell population.

2. Method as claimed in claim 1, **characterized in that** provision is made of at least 2, 3, 4, 5, or 6 selection reservoirs (12, 13, 14, 15, 16, 17; 30).

## Revendications

1. Procédé de sélection *in vitro* d'au moins une sous-population cellulaire thérapeutique à partir d'une population cellulaire originale, dans lequel la population cellulaire originale est sélectionnée parmi des cellules souches mésenchymateuses et des astroglies, dans lequel la sous-population cellulaire thérapeutique est utilisable pour la thérapie de maladies de tissus ou d'organes, et dans lequel le procédé présente les étapes suivantes:
a) placer une population cellulaire originale dans un premier réservoir initial (20), qui est raccordé à au moins un second réservoir de sélection (12, 13, 14, 15, 16, 17; 30) par au moins un moyen de sélection (18; 19) présentant au moins une ouverture (19; 29), dans lequel ladite au moins une ouverture (19; 29) dudit au moins un moyen de sélection (18; 19) présente un diamètre compris entre 8 µm et 12 µm;
b) placer au moins un milieu de culture de cellules dans ledit au moins un réservoir de sélection (12, 13, 14, 15, 16, 17; 30), vers lequel la sous-population cellulaire thérapeutique migre, dans lequel le milieu de culture de cellules est un milieu de culture spécifique au type de cellule sans additifs spécifiques, qui peuvent influencer la croissance ou la différentiation, l'activation et/ou la migration/ chimiotaxie des cellules;
c) cultiver la population cellulaire originale pendant un intervalle de temps de 15 minutes à 6 heures, intervalle de temps pendant lequel une sous-population cellulaire traverse ladite au moins une ouverture (19; 29) du moyen de sélection (18; 19) en direction dudit au moins un réservoir de sélection (12, 13, 14, 15, 16, 17; 30);
d) sélectionner et obtenir une cellule, qui est sélectionnée parmi au moins une des cellules qui ont traversé au moins une ouverture (19; 29) du moyen de sélection (18; 19) à l'intérieur d'un intervalle de temps des trente premières minutes comme première à centième cellule dans ledit au moins un réservoir de sélection (12, 13, 14, 15, 16, 17; 30), pour obtenir une sous-population cellulaire avec un potentiel de migration accru par rapport à la population cellulaire originale.

2. Procédé selon la revendication 1, **caractérisé en ce qu'**il est prévu au moins 2, 3, 4, 5 ou 6 réservoirs de sélection (12, 13, 14, 15, 16, 17; 30).
